# FASCICULE DE BREVET EUROPEEN

(11) **EP 1 525 047 B1**
(45) Date de publication et mention de la délivrance du brevet: **23.09.2009**
(21) Numéro de dépôt: 03755612.3
(22) Date de dépôt: 16.07.2003
(51) Int. Cl.: B01F 11/00, B01L 3/14, C12N 15/10, F16H 23/04

(54) **APPAREIL POUR LA VIBRATION RAPIDE DE TUBES CONTENANT DES ECHANTILLONS**
VORRICHTUNG ZUR SCHNELLEN SCHWINGUNG VON PROBENRÖHRCHEN
DEVICE FOR FAST VIBRATION OF TUBES CONTAINING SAMPLES

(30) Priorité: 01.08.2002 FR 0209832; 25.02.2003 FR 0302308
(43) Date de publication de la demande: 27.04.2005
(73) Titulaire: Bertin Technologies S.A., 78180 Montigny-le-Bretonneux (FR)
(72) Inventeur: BOQUET, Jean, F-78610 Le Perray-en-Yvelines (FR); ESTEVE, Alain-Jean, F-78110 Le Vesinet (FR)
(74) Mandataire: Ramey, Daniel
(86) Numéro de dépôt international: PCT/FR2003/002253
(87) Numéro de publication internationale: WO 2004/012851

(56) Documents cités:
- DE-A- 3 437 804
- US-A- 4 125 335
- US-A- 5 024 114
- US-A- 5 567 050
- US-A- 5 639 160
- US-B1- 6 235 501
- PATENT ABSTRACTS OF JAPAN vol. 0180, no. 71 (C-1162), 7 février 1994 (1994-02-07) & JP 05 284961 A (TAITETSUKU KK), 2 novembre 1993 (1993-11-02)

## Description

L'invention concerne un appareil pour la vibration rapide de tubes contenant des échantillons, en particulier des échantillons biologiques, la vibration rapide des tubes réalisant un broyage des échantillons.

Il est en effet déjà connu de broyer des échantillons biologiques en enfermant les échantillons dans des tubes qui contiennent également des microbilles de verre ou de céramique et en soumettant les tubes fermés de façon étanche à une vibration axiale à fréquence élevée, par exemple d'environ 100 Hz, pendant une durée relativement courte, de l'ordre de 30 à 60 secondes par exemple.

Le document US-A-5,567,050 décrit un appareil pour l'exécution d'un tel procédé comprenant un plateau de support des tubes et des moyens d'entraînement du plateau en mouvement oscillatoire autour d'un centre de rotation. Les moyens d'entraînement comprennent un moteur électrique dont l'arbre de sortie est muni d'un manchon à surface cylindrique extérieure inclinée en oblique par rapport à l'axe de l'arbre de sortie du moteur. Le plateau est monté libre en rotation sur le manchon au moyen de roulements axialement alignés et il est associé à des moyens d'immobilisation en rotation, de telle sorte que quand le manchon est entraîné en rotation par le moteur, il fait osciller le plateau autour d'un centre de rotation qui est formé par l'intersection de l'axe de l'arbre du moteur et de l'axe de la surface extérieure cylindrique du manchon. Les tubes fixés à la périphérie du plateau à égale distance du centre de rotation sont ainsi déplacés en mouvement alternatif sensiblement curviligne. Théoriquement, la vitesse de rotation de l'arbre de sortie du moteur peut être comprise entre 3000 et 8000 tours par minute et les échantillons sont soumis à des accélérations linéaires de 150 à 400 g, pour leur broyage.

Cet appareil connu présente toutefois l'inconvénient d'un échauffement rapide des roulements qui servent au montage du plateau sur le manchon et qui supportent tous les efforts de mise en rotation du plateau, ce qui provoque une usure rapide de ces roulements et une réduction très sensible de leur durée de vie, l'échauffement des roulements se traduisant également par un échauffement du plateau qui se transmet aux tubes et aux échantillons contenus dans les tubes. En pratique, il n'est pas possible avec ce type d'appareil de faire osciller le plateau à une vitesse supérieure à 6000 tours par minute environ sans provoquer la destruction plus ou moins rapide de l'appareil. En outre, l'échauffement des roulements et du plateau oblige à attendre un certain temps entre deux cycles de broyage, pour laisser refroidir suffisamment le plateau et les roulements.

De plus, dans cet appareil connu, les tubes doivent être manipulés un par un pour être placés dans les logements prévus sur le plateau et pour en être retirés, et il faut également mettre en place et manoeuvrer à la main des moyens de blocage des tubes dans leurs logements du plateau, ce qui allonge fortement les durées de chargement et de déchargement de l'appareil et augmente d'autant les durées totales des cycles d'analyse des échantillons.

La présente invention a notamment pour but d'apporter une solution simple, efficace et économique à ce problème.

Elle a pour objet un appareil du type précité, qui permet la vibration rapide de tubes contenant des échantillons à analyser et qui a une durée de vie très supérieure à celle des appareils concurrents connus.

Elle a également pour objet un appareil du type précité, qui peut fonctionner sans dommage à des fréquences d'oscillation élevées, supérieures à celles des appareils concurrents connus.

Elle a encore pour objet un appareil de ce type, qui est plus simple et moins coûteux et aussi plus facile et plus rapide à utiliser que les appareils connus.

Elle propose à cet effet, un appareil pour la vibration rapide de tubes contenant des échantillons, en particulier biologiques, comprenant un plateau de support des tubes et des moyens d'entraînement du plateau en mouvement oscillatoire autour d'un centre de rotation situé sur l'axe de symétrie du plateau, **caractérisé en ce que** le plateau est supporté et centré sur une partie suspendue élastiquement de l'appareil au moyen de deux paliers concentriques montés l'un à l'intérieur de l'autre, l'un de ces paliers étant du type à rotule permettant le mouvement oscillatoire du plateau autour du centre de rotation.

Un avantage essentiel de l'appareil selon l'invention est que les moyens de support et de centrage du plateau n'ont pas à supporter d'efforts dus à la rotation du plateau de sorte que ces moyens peuvent être formés par des paliers, notamment par des roulements sans que ces derniers soient rapidement détériorés ou détruits lors du fonctionnement de l'appareil.

De préférence, les deux paliers sont des roulements à billes. Le premier de ces paliers comprend une cage radialement interne solidaire d'un embout cylindrique axial du plateau, et une cage radialement externe qui est solidaire en rotation de la cage radialement interne du second palier. La cage radialement externe du second palier est solidaire de la partie fixe de l'appareil.

Le premier palier est une rotule à billes, qui permet le mouvement oscillatoire du plateau autour du centre de rotation. Le second palier est un roulement à billes dont la cage interne tourne à grande vitesse avec la cage externe du premier palier, les cages interne du premier palier et externe du second palier étant fixes en rotation, et la cage interne du premier palier oscillant avec le plateau par rapport à la cage externe de ce premier palier.

L'embout cylindrique du plateau est, à son extrémité libre, relié par un troisième palier, tel qu'un roulement à billes du type rotulant, à un excentrique monté en bout d'un arbre moteur.

Pour accepter les dilatations thermiques, ou bien l'embout est monté axialement coulissant dans le troisième palier, ou bien le premier palier est monté axialement coulissant à l'intérieur du second.

Selon encore d'autres caractéristiques de l'invention, les tubes sont montés dans des logements prévus dans des paniers en forme de secteurs circulaires qui sont positionnés et fixés de façon amovible sur le plateau, et des moyens sont prévus pour maintenir les tubes dans leurs logements, ces moyens comprenant avantageusement un couvercle monté sur le plateau pour recouvrir les tubes placés dans leurs logements, et des moyens de blocage ou de verrouillage du couvercle dans sa position de maintien des tubes.

Dans un mode de réalisation préféré de l'invention, le couvercle est maintenu par dépression sur le plateau dans une position de blocage des tubes dans leurs logements. Pour cela, le couvercle délimite avec le plateau une chambre étanche, qui est reliée à une source de vide.

De plus, des moyens de verrouillage à encliquetage sont prévus sur le plateau pour permettre un positionnement angulaire et un verrouillage rapide et automatique du couvercle dans sa position de maintien des tubes dans leurs logements.

Avantageusement, les moyens de liaison de la chambre à la source de vide forment des moyens d'immobilisation du plateau en rotation.

Dans un premier mode de réalisation, le couvercle comporte des doigts radiaux à sa périphérie, chaque doigt radial s'appliquant élastiquement sur le bouchon d'un tube pour maintenir le tube dans son logement du plateau.

Dans une variante de réalisation, une couronne comporte à sa périphérie des orifices de réception de tubes et permet le chargement des tubes à l'extérieur de l'appareil. Ensuite, la couronne portant les tubes est posée sur le plateau et est recouverte du couvercle précité, fixé et maintenu par dépression comme précédemment indiqué.

Dans une autre variante, la couronne précitée comprend des logements borgnes de réception des tubes et un anneau en caoutchouc ou analogue est posé sur la couronne pour fermer ces logements de façon étanche quand le couvercle est mis en place sur la couronne, elle-même posée sur le plateau.

Ainsi, en cas de rupture d'un tube au cours de la vibration, on évite une contamination globale de l'appareil et de son environnement.

Dans un mode de réalisation préféré de l'invention, les paliers concentriques du plateau sont portés par une couronne qui est fixée à un châssis fixe par des moyens de suspension élastique.

Le moteur électrique d'entraînement du plateau est monté fixement sur ce châssis, son arbre de sortie est relié par un accouplement élastique à un disque portant le troisième palier excentré d'entraînement de l'embout cylindrique du plateau, et ce disque est monté dans une boîte à roulements portée par le châssis.

On réalise ainsi un découplage entre le moteur, le disque portant le palier excentré et le plateau, ce qui évite d'appliquer au moteur les vibrations et contraintes de la couronne, du plateau et du disque. On prolonge ainsi la durée de vie du moteur et on peut faire fonctionner l'appareil à pleine charge à une plus grande vitesse de rotation du moteur.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue schématique en coupe axiale d'un mode de réalisation d'un appareil selon l'invention, dont le couvercle est ôté ;
- la figure 2 est une vue schématique en coupe axiale du plateau de l'appareil de la figure 1 avec son couvercle ;
- la figure 3 représente des moyens d'immobilisation en rotation ;
- la figure 4 est une vue schématique partielle des moyens de réception des tubes ;
- la figure 5 est une vue schématique en coupe d'une variante de réalisation de l'invention ;
- la figure 6 est une vue schématique partielle représentant une autre caractéristique de l'invention.

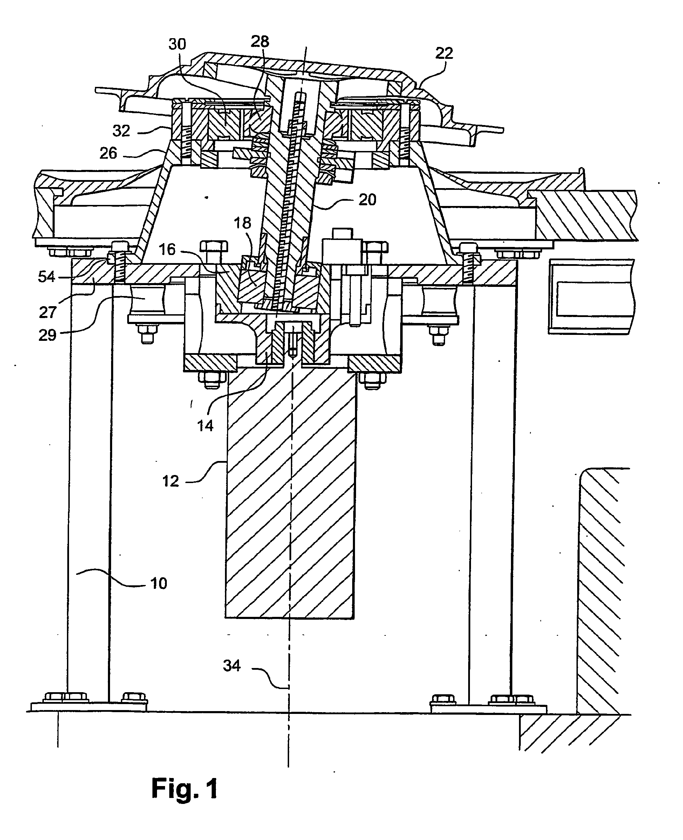

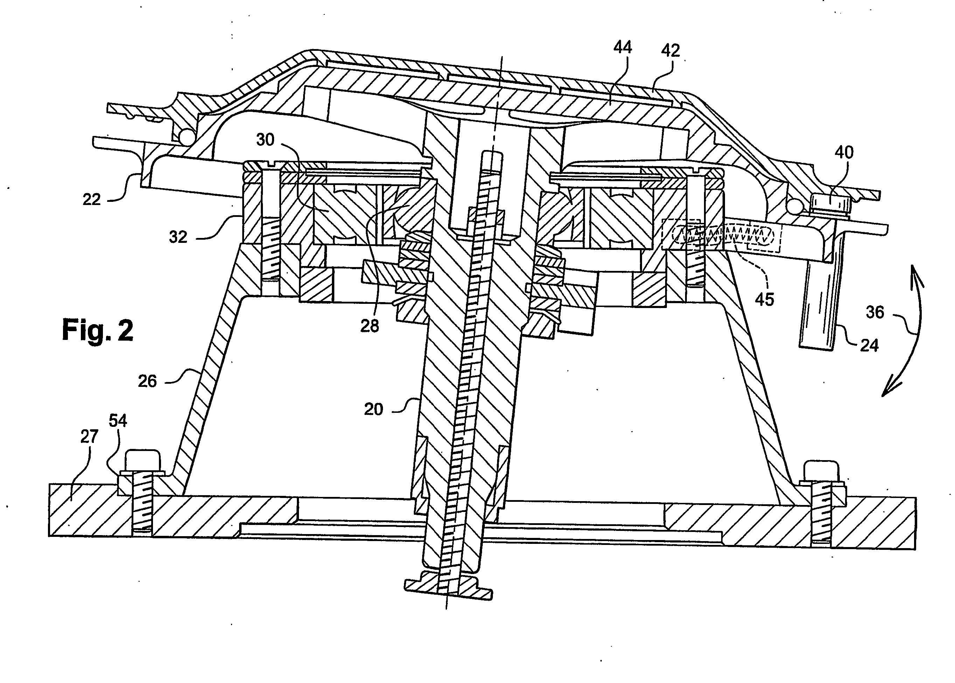

Dans le mode de réalisation représenté en figures 1 et 2, l'appareil selon l'invention comprend un châssis 10 dont la partie inférieure contient un moteur électrique 12 dont l'arbre de sortie 14 est orienté verticalement vers le haut et porte un disque 16 sur lequel un roulement 18 est monté oblique et de façon excentrée. Ce roulement 18 reçoit l'extrémité inférieure d'un arbre oblique 20 formé par un embout axialement cylindrique d'un plateau 22 de support de tubes à échantillons 24, le plateau 22 s'étendant perpendiculairement à l'arbre 20 et étant monté fixement en bout de celui-ci ou étant formé d'une pièce avec l'arbre 20, comme représenté. Le roulement 18 est incliné pour être axialement aligné avec l'arbre 20 et est de préférence du type rotulant pour le rattrapage des petits défauts mécaniques.

Le plateau 22 est supporté et centré sur une couronne 26 portée par le châssis 10, au moyen de deux paliers concentriques 28, 30 montés l'un à l'intérieur de l'autre entre l'arbre 20 et la couronne 26 et représentés de façon schématique aux figures 1 et 2.

Le premier palier 28 est une rotule à billes et comprend une cage radialement interne fixée sur l'arbre 20, et une cage radialement externe par rapport à laquelle la cage interne peut osciller autour d'un centre de rotation situé sur l'axe de l'arbre 20. Ce centre de rotation est le centre du mouvement oscillatoire du plateau 22 dans l'appareil selon l'invention.

La cage externe du palier 28 est solidaire en rotation de la cage interne du second palier 30, à l'intérieur de laquelle elle est montée.

La cage externe du second palier 30 est fixe à l'intérieur d'un anneau 32 qui est fixé par des vis sur la couronne 26. Cet anneau porte sur sa face supérieure une rondelle recouvrant tes paliers 28, 30 et les séparant de façon étanche de la partie supérieure du plateau 22 qui porte les tubes 24.

La couronne 26 comporte une bride périphérique 54 fixée par des vis sur une platine 27 montée sur le châssis 10 par des moyens de suspension élastique 29. Le moteur 12 est fixé sur le platine 27.

Des moyens sont prévus pour immobiliser le plateau en rotation et comprennent par exemple un ou plusieurs ressorts reliant radialement la couronne 26 à l'arbre 20.

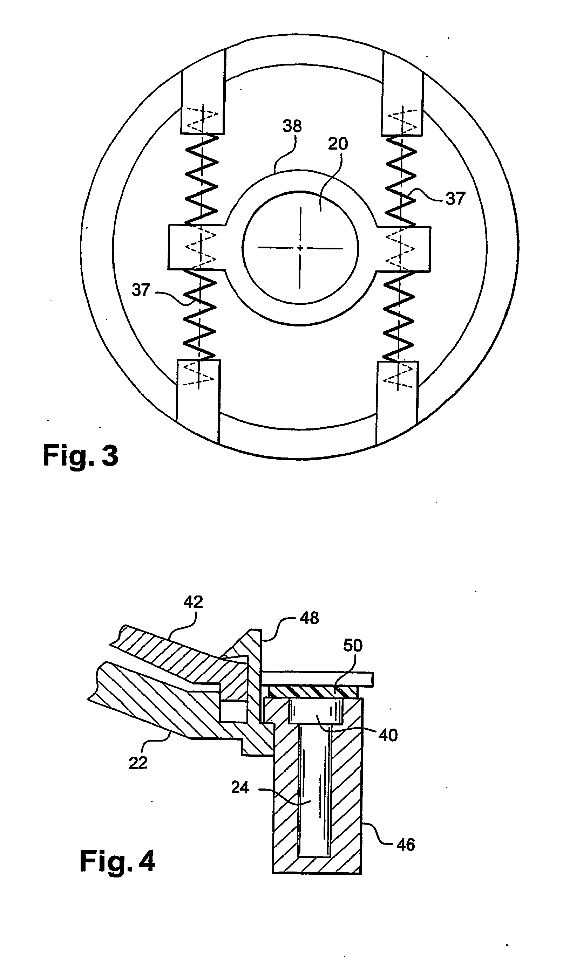

Dans un mode de réalisation particulier représenté schématiquement en figure 3, ces moyens comprennent deux ressorts 37 parallèles montés entre la couronne 26 et une rondelle 38 solidaire de l'arbre 20. Les parties médianes et les extrémités des ressorts sont fixées par vissage sur des pattes ou manchons cylindriques du socle 26 et de la rondelle 38 comme représenté. Quand l'arbre 20 et le plateau 22 ont tendance à tourner dans un sens, cela comprime deux moitiés de ressort 37 diamétralement opposées et détend les deux autres moitiés de ressort 37. Ces ressorts peuvent être faiblement précontraints en compression ou en traction dans la position d'équilibre représentée au dessin.

Le fonctionnement de cet appareil est le suivant :

Quand le moteur 12 est alimenté, le disque 16 est entraîné en rotation autour d'un axe vertical 34 et l'arbre oblique 20 tourne en parcourant une surface conique dont le sommet est à l'intersection entre l'axe vertical 34 de rotation du disque 16 et l'axe de l'arbre oblique 20. Le plateau 22 qui est fixe en rotation autour de l'axe de l'arbre oblique 20 et de l'axe vertical 34, est alors entraîné en mouvement oscillant autour du centre de rotation formé par l'intersection de l'axe 34 et de l'axe de l'arbre 20, et les tubes 24 portés par le plateau 22 sont déplacés en mouvement alternatif curviligne comme indiqué par la flèche 36.

Les cages externes du premier palier 28 et interne du second palier 30, fixes l'une par rapport à l'autre, sont entraînées en rotation à grande vitesse quand le disque 16 est entraîné en rotation. Cette rotation des cages n'intéresse que des pièces à faible inertie et presque sans frottement et n'absorbe qu'une énergie relativement faible. Grâce à la rotation de ces deux cages, le couple de rotation transmis au plateau 22 est relativement très faible, et le plateau peut être immobilisé en rotation par des moyens simples et légers.

Les deux paliers 28, 30 de l'appareil ne présentent aucun signe de fatigue ou d'usure lorsque le disque 16 est entraîné à des vitesses de rotation de l'ordre de 6500 tours par minute pendant des cycles de 60 secondes répétés à quelques minutes d'intervalles sur des durées de plusieurs jours. L'appareil selon l'invention est utilisable avec des vitesses de rotation de 8000 tours par minute, les tubes étant soumis à des accélérations de 600 g environ.

De plus, on peut modifier la course des tubes 24 simplement en changeant l'excentricité du roulement 18 ou le diamètre du plateau 22.

Les tubes 24 contenant les échantillons sont fermés par des bouchons 40 que l'on maintient en place au moyen d'un couvercle 42, de forme circulaire, qui est engagé sur la face supérieure du plateau 22. Le couvercle 42 comprend des doigts radiaux d'appui élastique sur les bouchons 40 des tubes 24 placés dans leurs logements qui sont formés par des orifices de la périphérie du plateau 22.

De plus, le couvercle 42 posé sur le plateau 22 délimite avec celui-ci une chambre 44 étanche qui est reliée à une source de vide extérieure à l'appareil par des conduits souples 45 qui relient le plateau 22 à la couronne 26 et qui peuvent avantageusement former des moyens d'immobilisation du plateau 22 en rotation. Les conduits souples 45 sont avantageusement logés à l'intérieur de ressorts à spires pour résister aux efforts et aux fréquences d'entraînement et assurer l'immobilisation du plateau 22 en rotation à la place des moyens 37, 38 décrits ci-dessus.

En outre, on prévoit sur le plateau 22 des doigts d'encliquetage élastique, qui s'étendent vers le haut et qui passent dans des orifices du couvercle 42 pour un positionnement angulaire et un verrouillage rapide et automatique du couvercle sur le plateau, ces doigts étant par exemple au nombre de deux et diamétralement opposés.

Une variante de réalisation représentée schématiquement en figure 4 permet à un opérateur de préparer individuellement les tubes 24, de les fermer au moyen des bouchons 40 et de les disposer ensuite dans leurs logements d'une couronne 46 amovible destinée à être montée entre le plateau 22 et le couvercle 42.

Quand la couronne est entièrement garnie de tubes 24, elle est chargée sur le plateau 22 de l'appareil de vibration. Le couvercle 42 est mis en place et verrouillé par encliquetage sur les doigts 48 du plateau puis maintenu par dépression dans la chambre 44, après quoi le moteur 12 est alimenté pour faire osciller le plateau 22 autour du centre de rotation précité.

A la fin d'un cycle de broyage, lorsque le moteur 12 est arrêté, la chambre 44 est mise à la pression ambiante, les doigts d'encliquetage sont repoussés élastiquement, le couvercle est retiré et la couronne 46 est retirée du plateau 22 et remplacée par une autre couronne garnie de tubes 24 contenant des échantillons à broyer.

Le chargement et le déchargement de l'appareil selon l'invention s'effectuent donc de façon simple et rapide.

Avantageusement, les logements de réception des tubes 24 dans la couronne 46 sont borgnes et une garniture annulaire 50 en caoutchouc ou analogue peut être posée sur ces logements pour les fermer individuellement de façon étanche.

Si un tube 24 est cassé au cours d'un cycle de vibration, son contenu est maintenu dans le logement correspondant du panier par la garniture en caoutchouc qui ferme ce logement de façon étanche. On évite ainsi une contamination globale de l'appareil et de son environnement.

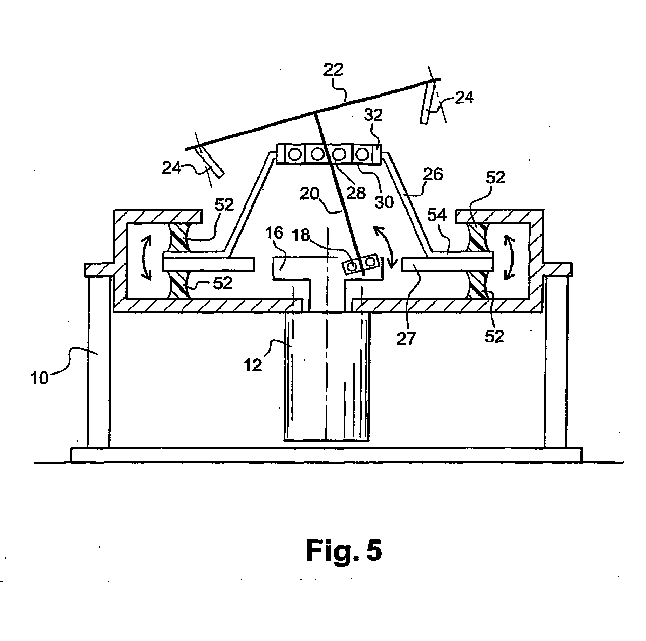

Dans le mode de réalisation préféré de l'invention, comme représenté schématiquement en figure 5, le moteur électrique 12 est fixé par des vis ou analogues directement sur le châssis 10 et la couronne 26 est montée sur le châssis 10 par des moyens de suspension élastique d'un type classique, qui comprennent par exemple des plots 52 en caoutchouc ou analogue reliés par paires au châssis 10 et à une bride périphérique 54 de la couronne 26 appliquée sur la platine 27.

Ce montage évite de transmettre au moteur 12 les vibrations et les efforts appliqués à la couronne 26, ce qui augmente de façon sensible la durée de vie du moteur.

De plus, on prévoit selon l'invention de monter les tubes à échantillons 24 sur le plateau 22 non plus parallèlement à l'axe de ce plateau, mais de façon inclinée en direction de cet axe, les axes des tubes 24 convergeant vers un point de l'axe de l'embout 20 du plateau, situé au dessus ou au dessous du plateau.

L'angle formé par les axes des tubes 24 avec l'axe de l'embout 20 est faible, typiquement compris entre 5 et 30° environ et de préférence de 10° environ.

En fonctionnement, les billes contenues avec les échantillons dans les tubes inclinés 24 sont mieux déplacées à travers les échantillons et réalisent un meilleur broyage de ceux-ci.

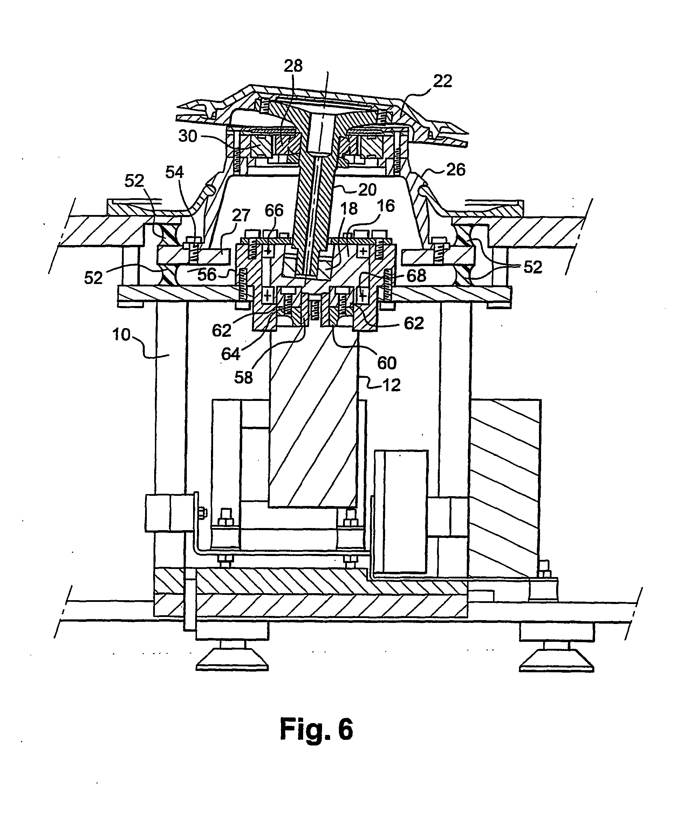

Une autre caractéristique avantageuse de l'invention est représentée en figure 6.

Dans cette figure, on retrouve le plateau 22 monté par les roulements 28 et 30 sur la couronne 26, elle-même montée sur le châssis 10 par la platine 27 et par les moyens 52 de suspension élastique.

L'embout 20 du plateau 22 est monté axialement coulissant à son extrémité inférieure dans le roulement excentré 18 porté par le disque 16 comme déjà décrit.

Le disque 16 n'est pas fixé directement sur l'arbre de sortie du moteur 12, mais est monté dans une boîte à roulements 56 fixée au châssis 10, et est entraîné en rotation par l'arbre du moteur 12 au moyen d'un accouplement élastique.

Cet accouplement élastique comprend un moyeu expansible 58 de montage d'une embase métallique 60 sur l'arbre de sortie du moteur 12, et des tubes de caoutchouc 62 qui sont reçus dans des logements de la base du disque 16 et fixés à l'embase 60 par des vis 64 logées dans les tubes 62.

La boîte à roulements 56 comprend un roulement horizontal supérieur 66 et un roulement horizontal inférieur 68, dont les cages extérieures sont bloquées dans la boîte 56. La cage intérieure du roulement supérieur 66 est solidaire du disque 16, qui peut coulisser dans la cage intérieure du roulement inférieur 68 pour des raisons de dilatation thermique.

Ce montage permet de mieux protéger encore le moteur 12 des vibrations et de le découpler au point de vue vibratoire du disque 16, du roulement 18 et de l'embout 20 du plateau 22. On augmente la durée de vie du moteur et l'appareil selon l'invention peut être utilisé à pleine charge à des vitesses de rotation plus élevées (par exemple 6800 tours/minute ou davantage au lieu de 6000 tours/minute) sans problèmes pour le moteur 12.

On notera enfin que cet appareil comprend avantageusement un capot pivotant qui vient coiffer le plateau 22 pour le broyage des échantillons et qui est équipé de moyens de verrouillage en position fermée du type électromagnétique ou à dépression.

## Revendications

1. Appareil pour la vibration rapide de tubes contenant des échantillons, en particulier biologiques, comprenant un plateau (22) de support des tubes (24), des moyens d'immobilisation du plateau en rotation autour de son axe et des moyens (12, 16, 18, 20) d'entraînement du plateau (22) en mouvement oscillatoire autour d'un centre de rotation situé sur l'axe du plateau, **caractérisé en ce que** le plateau (22) est supporté et centré sur une partie suspendue élastiquement (26) de l'appareil au moyen de deux paliers concentriques (28, 30) montés l'un à l'intérieur de l'autre, l'un de ces paliers étant du type à rotule permettant un mouvement oscillatoire du plateau autour du centre de rotation.

2. Appareil selon la revendication 1, **caractérisé en ce que** lesdits paliers (28, 30) sont des roulements à billes.

3. Appareil selon la revendication 1 ou 2, **caractérisé en ce que** un premier (28) desdits paliers comprend une cage radialement interne solidaire en rotation d'un embout axial (20) du plateau et une cage radialement externe solidaire en rotation d'une cage radialement interne du second (30) desdits paliers, dont la cage radialement externe est solidaire de ladite partie fixe de l'appareil.

4. Appareil selon la revendication 3, **caractérisé en ce que** le premier palier (28) est une rotule à billes.

5. Appareil selon la revendication 3 ou 4, **caractérisé en ce que** la cage externe du premier palier (28) est solidaire en rotation et en translation de la cage interne du second palier (30).

6. Appareil selon la revendication 3 ou 4, **caractérisé en ce que** la cage externe du premier palier (28) est mobile en translation axiale sur la cage interne du second palier (30).

7. Appareil selon l'une des revendications 1 à 6, **caractérisé en ce que** le plateau (22) est immobilisé en rotation autour de son axe par des moyens de liaison à ladite partie suspendue (26) de l'appareil.

8. Appareil selon l'une des revendications 1 à 7, **caractérisé en ce que** les moyens d'entraînement comprennent un troisième palier (18) excentré monté en bout d'un arbre moteur et reliant cet arbre moteur à un embout axial (20) du plateau.

9. Appareil selon la revendication 8, **caractérisé en ce que** l'embout (20) du plateau est mobile en translation axiale dans le troisième palier (18).

10. Appareil selon l'une des revendications précédentes, **caractérisé en ce que** les paliers concentriques (28,30) du plateau sont portés par une couronne (26) qui est montée sur un châssis fixe (10) par des moyens de suspension élastique (29, 52).

11. Appareil selon la revendication 10, **caractérisé en ce que** les moyens d'entraînement du plateau (22) comprennent un moteur électrique (12) monté fixement sur le châssis (10).

12. Appareil selon l'ensemble des revendications 8 et 11, **caractérisé en ce que** le troisième palier excentré (18) est porté par un disque (16) qui est monté dans une boîte à roulements (56) portée par le châssis (10) et qui est relié à l'arbre moteur (12) par un accouplement élastique (62, 64).

13. Appareil selon l'une des revendications précédentes, **caractérisé en ce que** les tubes (24) portés par le plateau (22) sont inclinés en direction de l'axe du plateau.

14. Appareil selon l'une des revendications précédentes, **caractérisé en ce que** le plateau (22) supporte une couronne circulaire (46), comportant des logements de réception des tubes (24) répartis de façon régulière autour du centre de rotation du plateau, un couvercle (42) de maintien des tubes (24) dans leurs logements, des moyens (44) d'application du couvercle (42) sur le plateau, et des moyens (48) de positionnement angulaire et de verrouillage du couvercle (42) dans sa position de maintien des tubes.

15. Appareil selon la revendication 14, **caractérisé en ce que** le couvercle (42) posé sur le plateau (22) délimite avec celui-ci une chambre (44) fermée de façon étanche, qui comporte des moyens de liaison à une source de vide.

16. Appareil selon la revendication 15, **caractérisé en ce que** les moyens de liaison à la source de vide forment des moyens d'immobilisation du plateau (22) en rotation autour de son axe.

17. Appareil selon l'une des revendications 14 à 16, **caractérisé en ce que** les moyens de positionnement angulaire et de verrouillage du couvercle sont des doigts (48) d'encliquetage élastique.

18. Appareil selon l'une des revendications 14 à 17, **caractérisé en ce qu'**une bande annulaire (50) d'élastomère ou analogue est interposée entre le couvercle et les sommets des tubes (24) et ferme à étanchéité les logements de la couronne (46) dans lesquels sont reçus les tubes (24).

## Claims

1. Apparatus for rapidly vibrating tubes containing samples, in particular biological samples, the apparatus comprising a disk (22) for supporting the tubes (24), means for preventing the disk from turning about its own axis, and means (12, 16, 18, 20) for driving the disk (22) in oscillating motion about a center of rotation situated on the axis of the disk, the apparatus being **characterized in that** the disk (22) is supported and centered on an elastically-suspended portion (26) of the apparatus by means of two concentric bearings (28, 30) mounted one within the other, one of the bearings being of the spherical type enabling the disk to execute oscillating motion about the center of rotation.

2. Apparatus according to claim 1, **characterized in that** said bearings (28, 30) are rolling bearings.

3. Apparatus according to claim 1 or claim 2, **characterized in that** a first of said bearings (28) has a radially-inner cage constrained to rotate with an axial endpiece (20) of the disk and a radially-outer cage constrained to rotate with a regularly inner cage of the second of said bearings (30), with its radially-outer cage being secured to the said stationary portion of the apparatus.

4. Apparatus according to claim 3, **characterized in that** the first bearing (28) is a spherical rolling bearing.

5. Apparatus according to claim 3 or claim 4, **characterized in that** the outer cage of the first bearing (28) is constrained to move in rotation and in translation with the inner cage of the second bearing (30).

6. Apparatus according to claim 3 or claim 4, **characterized in that** the outer cage of the first bearing (28) is movable in axial translation relative to the inner cage of the second bearing (30).

7. Apparatus according to any one of claims 1 to 6, **characterized in that** the disk (22) is prevented from turning about its axis by connection means connecting it to said suspended portion (26) of the apparatus.

8. Apparatus according to any one of claims 1 to 7, **characterized in that** the drive means comprise a third bearing (18) that is eccentric and mounted at the end of a drive shaft, connecting said drive shaft to an axial endpiece (20) of the disk.

9. Apparatus according to claim 8, **characterized in that** the endpiece (20) of the disk is movable in axial translation in the third bearing (18).

10. Apparatus according to any preceding claim, **characterized in that** the concentric bearings (28, 30) of the disk are carried by a ring (26) which is mounted on a stationary frame (10) by resilient suspension means (29, 52).

11. Apparatus according to claim 10, **characterized in that** the drive means of the disk (22) comprise an electric motor (12) mounted stationary relative to the frame (10).

12. Apparatus according to claims 8 and 11 taken together, **characterized in that** the eccentric third bearing (18) is carried by a turntable (16) which is mounted in a rolling-bearing box (56) carried by the frame (10) and which is connected to the drive shaft (12) via a resilient coupling (62, 64).

13. Apparatus according to any preceding claim, **characterized in that** the tubes (24) carried by the disk (22) are inclined towards the axis of the disk.

14. Apparatus according to any preceding claim, **characterized in that** the disk (22) supports a circular ring (46) having housings for receiving the tubes (24) distributed in regular manner around the center of rotation of the disk, a cover (42) for holding the tubes (24) in their housings, means (44) for holding the cover (42) against the disk, and means (48) for angularly positioning and locking the cover (42) in its tube-holding position.

15. Apparatus according to claim 14, **characterized in that** the cover (42) placed on the disk (22) co-operates therewith to define a chamber (44) that is closed in leaktight manner and that includes means for connection to a vacuum source.

16. Apparatus according to claim 15, **characterized in that** the means for connecting to the vacuum source forms means for preventing the disk (22) from turning about its axis.

17. Apparatus according to any one of claims 14 to 16, **characterized in that** the means for angularly positioning and locking the cover are resilient snap-fastening fingers (48).

18. Apparatus according to any one of claims 14 to 17, **characterized in that** an annular strip (50) of elastomer or the like is interposed between the cover and the tops of the tubes (24) to close in leaktight manner the housings in the ring (46) in which the tubes (24) are received.

## Patentansprüche

1. Vorrichtung zur schnellen Vibration von Proben enthaltenden Röhrchen, insbesondere biologischer Proben, umfassend eine Halterungsplatte (22) der Röhrchen (24), Mittel zur Immobilisierung der Platte gegenüber einer Drehung um ihre Achse, und Mittel (12, 16, 18, 20) zum Antrieb der Platte (22) in einer oszillierenden Bewegung um ein auf der Achse der Platte gelegenes Drehungszentrum, **dadurch gekennzeichnet, dass** die Platte (22) gehalten wird und zentriert ist auf einem elastisch aufgehängten Teil (26) der Vorrichtung mittels zweier konzentrischer Lager (28, 30), bei denen das eine innerhalb des anderen angeordnet ist, wobei eines der Lager vom Typ eines Drehlagers ist, was eine oszillierende Bewegung der Platte um das Drehungszentrum erlaubt.

2. Vorrichtung nach Anspruch 1, **dadurch gekennzeichnet, dass** die Lager (28, 30) Kugellager sind.

3. Vorrichtung nach Anspruch 1 oder 2, **dadurch gekennzeichnet, dass** ein erstes (28) dieser Lager ein radiales inneres Gehäuse umfasst, in der Drehung fest verbunden mit einem axialen Ansatzstück (20) der Platte, und ein radiales äußeres Gehäuse umfasst, in der Drehung fest verbunden mit einem radialen inneren Gehäuse des zweiten (30) Lagers, bei dem das radiale äußere Gehäuse fest verbunden mit dem feststehenden Teil der Vorrichtung ist.

4. Vorrichtung nach Anspruch 3, **dadurch gekennzeichnet, dass** das erste Lager (28) ein Kugellager ist.

5. Vorrichtung nach Anspruch 3 oder 4, **dadurch gekennzeichnet, dass** das äußere Gehäuse des ersten Lagers (28) in der Drehung und in der Translation fest verbunden mit dem inneren Gehäuse des zweiten Lagers (30) ist.

6. Vorrichtung nach Anspruch 3 oder 4, **dadurch gekennzeichnet, dass** das äußere Gehäuse des ersten Lagers (28) in der axialen Translation gegenüber dem inneren Gehäuse des zweiten Lagers (30) beweglich ist.

7. Vorrichtung nach einem der Ansprüche 1 bis 6, **dadurch gekennzeichnet, dass** die Platte (22) gegenüber einer Drehung um ihre Achse durch Verbindungsmittel am elastisch aufgehängten Teil (26) des Vorrichtungs immobilisiert ist.

8. Vorrichtung nach einem der Ansprüche 1 bis 7, **dadurch gekennzeichnet, dass** die Antriebsmittel ein drittes Lager (18) umfassen, das exzentrisch am Ende einer Antriebswelle angeordnet ist, und welches die Antriebswelle mit einem axialen Ansatzstück (20) der Platte verbindet.

9. Vorrichtung nach Anspruch 8, **dadurch gekennzeichnet, dass** das axiale Ansatzstück (20) der Platte in axialer Translation im dritten Lager (18) beweglich ist.

10. Vorrichtung nach einem der vorstehenden Ansprüche, **dadurch gekennzeichnet, dass** die konzentrischen Lager (28, 30) der Platte durch einen Kranz (26) getragen werden, der auf einem festen Gestell (10) durch elastische Aufhängungsmittel (29, 52) angeordnet ist.

11. Vorrichtung nach Anspruch 10, **dadurch gekennzeichnet, dass** die Antriebsmittel der Platte (22) einen elektrischen Motor (12) umfassen, der fest auf dem Gestell (10) angeordnet ist.

12. Vorrichtung nach den Ansprüchen 8 und 11, **dadurch gekennzeichnet, dass** das dritte exzentrische Lager (18) auf einer Scheibe (16) angeordnet ist, welche in einer Lagerbüchse (56) angeordnet ist, welcher am Gehäuse (10) angeordnet ist und welcher mit der Antriebswelle (12) über eine elastische Kupplung (62, 64) verbunden ist.

13. Vorrichtung nach einem der vorstehenden Ansprüche, **dadurch gekennzeichnet, dass** die Röhrchen (24), die an der Platte (22) angeordnet sind, zur Richtung der Achse der Platte geneigt sind.

14. Vorrichtung nach einem der vorstehenden Ansprüche, **dadurch gekennzeichnet, dass** die Platte (22) einen kreisförmigen Kranz trägt, der Plätze für die Aufnahme der Röhrchen (24) aufweist, welche in regelmäßiger Weise um das Drehungszentrum der Platte aufgeteilt sind, einen Deckel (42) zum Halten der Röhrchen (24) in ihren Plätzen, Mittel (44) zum Ausbringen des Deckels auf die Platte, und Mittel (48) zur Winkelpositionierung und zur Verriegelung des Deckels in seiner Röhrchen-haltenden Position.

15. Vorrichtung nach Anspruch 14, **dadurch gekennzeichnet, dass** der Deckel (42), der auf der Platte (22) angeordnet ist, mit dieser eine Kammer (44) begrenzt, die in einer luftdichten Weise verschlossen ist, welche Mittel zur Verbindung mit einer Vakuumquelle aufweist.

16. Vorrichtung nach Anspruch 15, **dadurch gekennzeichnet, dass** die Mittel zur Verbindung mit der Vakuumquelle Mittel zur Immobilisierung der Platte (22) gegenüber einer Drehung um ihre Achse bilden.

17. Vorrichtung nach einem der Ansprüche 14 bis 16, **dadurch gekennzeichnet, dass** die Mittel zur Winkelpositionierung und derVerriegelung des Deckels elastische Rastfinger (48) sind.

18. Vorrichtung nach einem der Ansprüche 14 bis 17, **dadurch gekennzeichnet, dass** ein ringförmiges Band (50) aus einem Elastomer oder Ähnlichem zwischen den Deckel und die Enden der Röhrchen (24) gebracht wird, und die Plätze des Kranzes (46), in denen die Röhrchen (24) aufgenommen sind, dicht verschließt.
